# EUROPEAN PATENT APPLICATION

(11) **EP 3 854 923 A1**
(43) Date of publication of application: **28.07.2021**
(21) Application number: 20153066.4
(22) Date of filing: 22.01.2020
(51) Int. Cl.: D04H 1/425, D04H 1/46, D04H 1/492, D04H 1/587, D04H 1/64, A61K 8/02, A47L 13/16

(54) **BIODEGRADABLE NON-WOVEN FABRIC AND METHOD FOR PRODUCING THE SAME**

(71) Applicant: Glatfelter Gernsbach GmbH, 76593 Gernsbach (DE)
(72) Inventor: Röttger, Henning, 24568 Kaltenkirchen (DE)
(74) Representative: Dilg, Haeusler, Schindelmann Patentanwaltsgesellschaft mbH

(57) **Abstract**

The present invention relates to a biodegradable non-woven fabric, a method for producing a biodegradable non-woven fabric and a wipe. The biodegradable non-woven fabric comprises biodegradable fibers. At least a part of the biodegradable fibers is entangled with each other, such that individual biodegradable fibers are in contact with each other at entangling points. At least a part of the entangling points is provided with a biodegradable binder.

## Description

### FIELD OF THE INVENTION

The present invention relates to a biodegradable non-woven fabric, a method for producing a biodegradable non-woven fabric and wipes comprising the biodegradable non-woven fabric. In particular, the biodegradable non-woven fabric may be a compostable, (substantially) plastic free spunlace wipe substrate (containing only plastic free fibers as fibers) with enhanced mechanical properties, such as tensile strength and resiliency.

### BACKGROUND

Disposable wipes, such as wet toilet wipes, baby wipes or other personal care wipes, are very popular for cleaning the skin of human bodies or facilities in the household because of their comfort for consumers and efficacy in cleaning. However, increasing concerns about plastic contamination of the environment create an increasing demand for plastic-free and fully compostable/biodegradable substrates for disposable wipes and similar single use products.

Spunlacing (which may also be referred to as hydroentanglement) and needle punching are technologies conventionally used for producing non-woven fabrics or substrates suitable as wipes, top-sheet and backsheet materials in hygiene and medical applications and as soft nonwovens with high CD elongation used in elastic laminates. Spunlacing is a bonding process for wet or dry fibrous webs where fine, high pressure jets of water penetrate the fibrous web and cause an entanglement of fibers, thereby providing fabric integrity. Needle punching is a bonding process where fibers are mechanically intertwined by needles. Spunlacing and needle punching create a textile structure by creating bonding points in a lose layer of fibers without the application of glue or binder fibers resulting in materials with very high softness and drapeability.

Well-established compostable substrates for applications like baby-wipes are spunlace materials only containing compostable fibers like viscose, tencel and cotton. Due to the avoidance of binders in the spunlace process, the biodegradability is only dependent on the choice of fibers. The use of such wipes substrates so far has been very limited because of the significantly higher cost of fibers like viscose and tencel compared to synthetic PET fibers commonly used in baby-wipes and similar products blended with viscose or tencel fibers.

Wetlaid/airlaid combined with spunlace technology processing a blend of pulp and cellulosic fibers such as viscose or tencel fibers as it is commonly used for the production of dispersible wipes (moist toilet tissue) is facing two limitations for using such substrates in applications like baby wipes requiring higher mechanical strength:
(i) The material strength is low compared to standard spunlace materials even if the fiber content is increased compared to the recipe used for dispersible wipes. As the pulp fibers are too short and too stiff to be entangled by the hydroentangling process, they are only entrapped in the structure of the hydroentangled viscose/tencel fibers but do not contribute to the mechanical strength of the web. Therefore, such materials require a significantly increased basis weight compared to standard spunlace to achieve similar mechanical strength resulting in unfavorable cost.
(ii) As the pulp fibers are only entrapped within the structure of hydroentangled viscose/tencel fibers and do not have bonding points themselves connecting the pulp fiber to one another or to the viscose/tencel fibers, they can move and clump together especially after exposed to liquid/water and get exposed to mechanical stress (crumpling of the web) destroying the textile structure of the web. Compared to spunlace and airlaid materials where all fibers are connected by bonding points creating a 3-dimensional structure preserving a textile like structure even after exposure to liquid/water and mechanical stress, this behavior is considered by consumers as clumping similar to standard toilette tissue".

Chemical bonded airlaid (LBAL) using biodegradable dispersion binders are used for compostable table-top materials but have not been successful as wet wipe because of the too high material stiffness resulting from the properties of the available binders and the high amount of binder required to achieve the required mechanical properties.

Thus, the hitherto known technologies face challenges on cost and/or technical performance.

### OBJECTS OF THE INVENTION

The present invention aims at overcoming the above described problems and drawbacks. Thus, it may be an object of the present invention to provide a biodegradable non-woven fabric suitable for disposable applications, such as wipes, with enhanced mechanical properties such as tensile strength and resiliency of the spunlaced or needle punched fabric or substrate allowing to reduce the basis weight of the substrates, thereby saving material cost.

### SUMMARY OF THE INVENTION

The present inventor has made diligent studies for solving these objects and has found that by applying a biodegradable (in particular hydrophilic) binder to spunlace materials substantially only containing biodegradable fibers in-line between spunlace unit and dryer (in particular a through air dryer) without fully soaking the web, the binder may preferably move to the entangling points of the spunlace material especially if the excessive water is removed from the web after the spunlace section prior to applying the binder and drying/curing the web. Without wishing to be bound by any theory, the present inventor assumes that this preferred movement of the binder to the entangling points may be via capillary force due to the condensed structure (lower pore size) in the entangling points of the spunlace material. The preferred accumulation of the binder in the entangling points providing a low fiber to fiber distance may help to create bonding points between the fibers and may coat the surface of the fibers thereby increasing surface friction. The increase of surface friction and/or creating of inter-fiber bonding points may increase the strength of the entangling points avoiding fibers slipping out of these entangling points by application of mechanical stress. Minimizing the inter-fiber movement in the entangling points may also allow to adjust the elongation of a spunlace material improving the puncture resistance and/or resiliency of the material.

By (preferably or selectively) providing the entangling points of an entangled fabric, the mechanical strength (especially puncture resistance) and/or resiliency of spunlace or needle punched materials substantially only containing biodegradable fibers may be increased allowing to reduce the basis weight of wipes substrates saving material cost. In particular, the reduction of basis weight may enable the use of more expensive biodegradable fibers replacing synthetic fibers like PET while nevertheless keeping the unit cost ($/m²) in a range required for cost sensitive applications like baby wipes. In addition, by moving the binder to the entangling points, thereby minimizing the binder content in the less condensed areas in-between, the favorable mechanical properties (such as cushiness, softness, bulk, and the like) of the spunlace material may be preserved or even enhanced.

As a result, a biodegradable non-woven fabric suitable for disposable applications, such as wipes, with increased mechanical strength and/or resiliency of the spunlaced or needle punched fabric or substrate allowing to reduce the basis weight of the substrates, thereby saving material cost, may be obtained.

Accordingly, the present invention relates to a biodegradable non-woven fabric comprising biodegradable fibers, wherein at least a part of the biodegradable fibers is entangled with each other, such that individual biodegradable fibers are in contact with each other at entangling points, and wherein at least a part of the entangling points is (selectively) provided (or enriched) with a biodegradable binder (such that surface friction between the biodegradable fibers at the entangling points is increased or inter-fiber bondings are formed, thereby fixing the entangling points).

The present invention further relates to a method for producing a biodegradable non-woven fabric, comprising the steps of (a) forming a fibrous web comprising biodegradable fibers, (b) entangling at least a part of the biodegradable fibers with each other by subjecting the fibrous web to a water-jet treatment or needle punching, (c) applying a biodegradable binder (for instance in the form of a solution or dispersion) to the entangled fibrous web; and (d) drying the entangled fibrous web (thereby curing the binder).

In addition, the present invention relates to a biodegradable non-woven fabric obtainable by a method for producing a biodegradable non-woven fabric as described herein.

Moreover, the present invention relates to a wipe comprising or consisting of the biodegradable non-woven fabric as described herein.

Other objects and many of the attendant advantages of embodiments of the present invention will be readily appreciated and become better understood by reference to the following detailed description of embodiments.

### DETAILLED DESCRIPTION OF THE INVENTION

Hereinafter, details of the present invention and other features and advantages thereof will be described. However, the present invention is not limited to the following specific descriptions, but they are rather for illustrative purposes only.

It should be noted that features described in connection with one exemplary embodiment or exemplary aspect may be combined with any other exemplary embodiment or exemplary aspect, in particular features described with any exemplary embodiment of a biodegradable non-woven fabric may be combined with any exemplary embodiment of a method for producing a biodegradable non-woven fabric or with any exemplary embodiment a wipe and vice versa, unless specifically stated otherwise.

Where an indefinite or definite article is used when referring to a singular term, such as "a", "an" or "the", a plural of that term is also included and vice versa, unless specifically stated otherwise, whereas the word "one" or the number "1", as used herein, typically means "just one" or "exactly one".

The expression "comprising", as used herein, includes not only the meaning of "comprising", "including" or "containing", but also encompasses "consisting essentially of" and "consisting of".

Unless specifically stated otherwise, the expression "at least a part of", as used herein, may mean at least 5 % thereof, in particular at least 10 % thereof, in particular at least 15 % thereof, in particular at least 20 % thereof, in particular at least 25 % thereof, in particular at least 30 % thereof, in particular at least 35 % thereof, in particular at least 40 % thereof, in particular at least 45 % thereof, in particular at least 50 % thereof, in particular at least 55 % thereof, in particular at least 60 % thereof, in particular at least 65 % thereof, in particular at least 70 % thereof, in particular at least 75 % thereof, in particular at least 80 % thereof, in particular at least 85 % thereof, in particular at least 90 % thereof, in particular at least 95 % thereof, in particular at least 98 % thereof, and may also mean 100 % thereof.

In a first aspect, the present invention relates to a biodegradable non-woven fabric.

The term "non-woven fabric", as used herein, may in particular mean a web of individual fibers which are intertwined, but not in a regular manner as in a knitted or woven fabric.

The term "biodegradable" (which may also be referred to as "compostable"), as used herein, may in particular mean that the material concerned, such as the biodegradable non-woven fabric, the biodegradable fibers, the biodegradable binder and the like, complies with the requirements for industrial compostability, for instance in accordance with EN 13432, and preferably also with the requirements for home compostability.

The biodegradable non-woven fabric comprises biodegradable fibers.

In an embodiment, the biodegradable fibers comprise cellulosic fibers. The term "cellulosic fibers", as used herein, may in particular denote fibers based on cellulose, in particular modified or regenerated cellulose fibers, such as fibers prepared from cellulose, or cellulose derivates, such as ethyl cellulose, cellulose acetate and the like. The term "regenerated cellulose fibers", as used herein, may in particular denote manmade cellulose fibers obtained by a solvent spinning process.

In an embodiment, the regenerated cellulose fibers may be selected from the group consisting of viscose (rayon) or lyocell (tencel).

Viscose is a type of solvent spun fiber produced according to the viscose process typically involving an intermediate dissolution of cellulose as cellulose xanthate and subsequent spinning to fibers.

Lyocell is a type of solvent spun fiber produced according to the aminoxide process typically involving the dissolution of cellulose in N-methylmorpholine N-oxide and subsequent spinning to fibers.

Moreover, the biodegradable fibers may also comprise thermoplastic biodegradable fibers, for instance made of polylactic acid (PLA) and/or polybutylene succinate (PBS). Also bicomponent fibers made of thermoplastic biodegradable materials may be used, such as PLA/PLA bicomponent fibers or PLA/PBS bicomponent fibers. The term "thermoplastic fibers", as used herein, may in particular denote fibers that soften and/or partly melt when exposed to heat and are capable to bind with each other or to other non-thermoplastic fibers, such as cellulose fibers, upon cooling and resolidifying (Thermobonding).

In an embodiment, the biodegradable fibers have an average fiber length of from 15 to 60 mm, for instance an average fiber length of from 18 to 50 mm, such as an average fiber length of from 20 to 40 mm.

In an embodiment, the biodegradable fibers may have a fiber coarseness of from 0.5 to 4.0 dtex, such as of from 1.0 to 2.0 dtex.

In the biodegradable non-woven fabric, at least a part of the biodegradable fibers is entangled with each other.

The term "entangled", as used herein, may in particular mean that the biodegradable fibers are at least partly intertwined with each other, thereby imparting strength, such as tear strength or tensile strength, to the non-woven fabric. Entangling of the biodegradable fibers might in particular be achieved by a treatment of a fibrous web with water jets, as will be explained in further detail below, which may also be referred to as "hydroentanglement" or "spunlacing" and the entangled fibers may thus also be referred to as "hydroentangled fibers" or "spunlaced fibers". Alternatively, entangling of the biodegradable fibers might in particular be achieved by needle punching where the biodegradable fibers are mechanically intertwined by means of needles.

In the biodegradable non-woven fabric, the at least a part of the biodegradable fibers is entangled with each other such that individual biodegradable fibers are in contact with (or at least in close distance to) each other at entangling points. Thus, a structure of biodegradable fibers may be formed where an individual biodegradable fiber is in (physical) contact with (or at least in close distance to) one or more other biodegradable fibers at one or more positions or locations (which are referred to herein as "entangling points") along the length of the individual fiber, whereas other portions of the biodegradable fibers are not in contact with or in close distance to any other fiber (which portions may also be referred to as "areas between the entangling points" or "interstitial portions").

In the biodegradable non-woven fabric, at least a part of the entangling points is provided with a biodegradable binder. In particular, the biodegradable binder may be provided selectively at the at least a part of the entangling points (i.e. a relatively large amount of the biodegradable binder is provided at the at least a part of the entangling points compared to the interstitial portions or portions between the entangling points). As a result, the surface friction between the biodegradable fibers at the entangling points may be increased and/or inter-fiber bondings may be formed, thereby fixing the entangling points and/or increasing the strength of the entangled non-woven fabric resulting in an increased force required to disentangle or dislocate fibers in the nonwoven structure.

In an embodiment, the biodegradable binder may be enriched in (or at) the entangling points of the non-woven fabric whereas the amount of the binder is (substantially) lower in areas between the entangling points. For example, the amount of the binder may be lower by at least 25 %, in particular by at least 50 %, at the interstitial portions or the portions between the entangling points compared with the entangling points. As a result, the textile properties of the entangled non-woven fabric may be maintained.

Without wishing to be bound by any theory, the present inventor assumes that this enrichment of the biodegradable binder in (or at) the entangling points of the non-woven fabric is due to a movement of the binder, when applied to the entangled fibrous web, via capillary force because of the condensed structure (lower pore size) in the entangling points or via a preferred interaction between the binder molecules and the molecules of the biodegradable fibers.

In an embodiment, covalent bonding points are created between the biodegradable fibers preferably within entangled areas so that the entangling points may form (or act as) bonding points.

In an embodiment, the binder at least partially coats a surface of the biodegradable fibers especially in the entangled areas thereby increasing the surface friction between the fibers.

In an embodiment, the biodegradable binder further comprises glycerol or similar additives reducing the brittleness of the (dried treated) non-woven fabric. In other words, glycerol or similar additives reducing the brittleness of the non-woven fabric may be added to the biodegradable binder in order to reduce the brittleness of the dried treated non-woven fabric.

A particular characteristic of embodiments of the invention may be that a significant increase in strength (such as in machine direction and/or in cross-direction, in a dry state and/or in a wet state) and/or increase of resiliency (resulting in perceived bulkiness/loftiness of the material by a relatively low amount of biodegradable binder may be achieved in view of the (selective or enriched) provision of the binder at the entangling points. Moreover, the strength and/or bulkiness can be properly tailored to the actual needs by appropriately adjusting the applied amount of the biodegradable binder. Still further, since no non-biodegradable components or ingredients are required, the non-woven fabric may be (entirely) compostable, thereby contributing to the avoidance of environmental pollution with plastic materials.

The term "binder", as used herein, may in particular denote a chemical compound that is able to bind (e.g. by forming covalent bonds, by ionic interactions or the like) to two or more fibers, thereby interconnecting the fibers, resulting in an increased tensile strength and/or resiliency of the web or fabric.

In an embodiment, the biodegradable binder may be comprised in an amount of from 0.1 to 20 wt.-%, such as in an amount of from 0.2 to 10 wt.-%, such as in an amount of from 0.35 to 5 wt.-%, such as in an amount of from 0.5 to 2.5 wt.-%, based on the total weight of the non-woven fabric.

In an embodiment, the biodegradable binder is a hydrophilic binder, rather than a hydrophobic binder. The term "hydrophilic binder", as used herein, may in particular denote that the binder is one whose interactions with water and other polar substances are more thermodynamically favorable than its interactions with oil or other hydrophobic solvents.

For example, the biodegradable binder may be selected from the group consisting of chitosan, modified starch, cellulose derivatives, in particular blends of carboxymethylcellulose and citric acid, protein based binders, such as casein, and others. Combinations of two or more thereof may also be applied. Further suitable biodegradable binder are disclosed in WO 2014/117964 A1, the disclosure of which is incorporated herein by reference.

In an embodiment, the biodegradable non-woven fabric may have a grammage or basis weight of from 20 to 300 g/m², such as from 25 to 200 g/m², such as from 30 to 150 g/m², such as from 40 to 100 g/m², such as from 50 to 80 g/m².

In an embodiment, substantially all fibers comprised in the biodegradable non-woven fabric may be biodegradable fibers, in particular the biodegradable fibers described herein. In other words, it may be possible that the biodegradable non-woven fabric does substantially not comprise any other fibers than biodegradable fibers, in particular the biodegradable fibers described herein. With regard to embodiments comprising "substantially no other fibers than biodegradable fibers", other fibers than biodegradable fibers, if any, may still be present in relatively minor amounts of up to 10, up to 5, up to 3, up to 2, or up to 1 wt.-% based on the total weight of the non-woven fabric.

In an embodiment, the biodegradable non-woven fabric may further comprise pulp fibers, for instance up to 20, up to 10, up to 5, up to 3, up to 2, or up to 1 wt.-% based on the total weight of the non-woven fabric. The pulp fibers may be natural pulp fibers, in particular pulp fibers of natural origin, such as softwood pulp fibers or hardwood pulp fibers. Pulp may in particular denote a (lignocellulosic) fibrous material prepared by chemically or mechanically separating cellulose fibers from wood or the like, such as by a kraft process (sulfate process).

In an embodiment, the biodegradable non-woven fabric may substantially not comprise thermoplastic non-biodegradable (synthetic) fibers, such as polyethylene fibers, polypropylene fibers, polyester fibers (such as polyethylene terephthalate (PET) fibers), and the like. The term "non-biodegradable fibers", as used herein, may in particular denote fibers that do not comply with the requirements for industrial compostability, for instance in accordance with EN 13432. With regard to embodiments comprising "substantially no thermoplastic non-biodegradable (synthetic) fibers", thermoplastic non-biodegradable (synthetic) fibers, if any, may still be present in relatively minor amounts of up to 10, up to 5, or up to 2 wt.-% based on the total weight of the non-woven fabric.

In an embodiment, the non-woven fabric may be treated (impregnated) with a liquid or a lotion. In other words, the non-woven fabric may further comprise a liquid or a lotion. In such situation, the non-woven fabric may in particular represent a wet wipe. The liquid or the lotion is not particularly limited, and any liquid or lotion customary in the field of wet wipes may be applied. Typically, the liquid or the lotion may comprise a solvent, such as water, an alcohol, or mixtures thereof, surfactants or detergents, skin care agents, emollients, humectants, perfumes, preservatives etc. depending on the intended use.

In a second aspect, the present invention relates a method for producing a biodegradable non-woven fabric, in particular of a biodegradable non-woven fabric as described herein.

The method comprises the steps of:
(a) forming a fibrous web comprising biodegradable fibers;
(b) entangling at least a part of the biodegradable fibers with each other by subjecting the fibrous web to a water-jet treatment or needle punching;
(c) applying a biodegradable binder to the entangled fibrous web; and
(d) drying the entangled fibrous web.

In step (a), the fibrous web may be prepared by a carding or an airlaid process (dry forming) creating a layer of non-bonded fibers which is subsequently fed into a spunlacing (hydroentagling) or needle punching process creating a web with bonding points by entangled fibers having textile properties (web-strength, drape-ability, resiliency). In case of materials containing a certain amount of pulp fibers, these can be added either within the airlaid web forming step or by adding the the pulp fibers using an airlaid forming section behind the card or by adding a layer of tissue combined with the layer of fibers.

In step (b), at least a part of the biodegradable fibers are entangled with each other by subjecting the fibrous web to a water-jet treatment or needle punching.

The term "water-jet treatment", as used herein, may in particular mean a process of mechanically entangling fibers by giving the fibrous web an impact with jets of water. Water-jet treatment may also be referred to as hydroentanglement or spunlacing. Water-jet treatment typically involves the ejection of fine, high pressure jets of water from a plurality of nozzles on a fibrous web provided on a forming wire. The water jets penetrate the web, hit the belt where they may be reflected and pass again the web causing the fibers to entangle. Thus, by subjecting the fibrous web to the water-jet treatment, the fibers are entangled, in particular hydroentangled.

The term "needle punching", as used herein, may in particular mean a bonding or entanglement technique where the fibers are mechanically intertwined by means of needles. In particular, fine needles may repeatedly stitch into a fibrous web such that fibers become intertwined or entangled.

If at least a part of the biodegradable fibers are entangled with each other by subjecting the fibrous web to a water-jet treatment (rather than by needle punching), it might be advantageous to remove at least some of the (excessive) liquid (or water) used for spunlacing before applying a biodegradable binder to the entangled fibrous web. By taking this measure, a dilution of the binder may be avoided and also the preferred transport of liquid to the entangled zones by means of capillary force may be facilitated or promoted. The at least partial removal of (excessive) water may be achieved for instance by the application of vacuum (such as vacuum suction), application of pressure (such as by means of a gas stream, e.g. an "air-knife") or others.

In step (c), the biodegradable binder may be applied in the form of a solution or dispersion to the entangled fibrous web. For instance, the biodegradable binder may be applied by spraying or other means of liquid application like a size-press, a foulard or other.

In step (d), the drying of the entangled fibrous web may preferably be carried out such that the biodegradable binder is cured. In particular, the drying is preferably carried out at a (sufficiently high) temperature to cure the biodegradable binder, for instance at a temperature of more than 80 °C, such as more than 100 °C, such as more than 120 °C, such as more than 140°C, such as more than 180 °C.

In an embodiment, the drying comprises through-air drying, in particular involving hot air. By taking this measure, the movement of the applied (yet uncured) binder towards the entangling points and the accumulation of the binder at the entangling points due to the condensed structure (lower pore size) at the entangling points may be further promoted, which may result in a further enrichment of the binder at the entangling points. Furthermore, a compaction of the web may be avoided maintaining the bulk/loftiness of the web.

In a third aspect, the present invention relates to a biodegradable non-woven fabric obtainable by a method for producing a biodegradable non-woven fabric as described herein. In particular, a biodegradable non-woven fabric obtainable by a method for producing a biodegradable non-woven fabric as described herein may have any of the properties or features of a biodegradable non-woven fabric according to the first aspect, as described in the foregoing.

In a fourth aspect, the present invention relates to a wipe comprising or consisting of the biodegradable non-woven fabric as described herein. In particular, the non-woven fabric according to the present invention may be usable as a wipe.

In an embodiment, the wipe may be a dry wipe. Dry wipes may be particularly suitable for use as kitchen towel or surface cleaning substrate, enabling a rapid and efficient pickup of liquids.

In an embodiment, the wipe may be a wet wipe. For instance, the wet wipe may be treated with a liquid or a lotion, as described in further detail above. Wet wipes may be particularly suitable for cleaning the skin of a human body, including the private parts thereof. Thus, wet wipes may be particularly suitable for use as personal care wipes, facial wipes or baby wipes.

In an embodiment, the wipe is selected from the group consisting of facial wipes, cosmetic wipes, baby wipes, sanitary wipes, kitchen tissue, paper towel, handkerchiefs (facial tissue), cleaning tissue, cleansing tissue, floor mop and hard surface cleaning wipe.

While the present invention has been described in detail by way of specific embodiments, the invention is not limited thereto and various alterations and modifications are possible, without departing from the scope of the invention.

## Claims

1. A biodegradable non-woven fabric comprising biodegradable fibers,
wherein at least a part of the biodegradable fibers is entangled with each other, such that individual biodegradable fibers are in contact with each other at entangling points,
**characterized in that**
at least a part of the entangling points is provided with a biodegradable binder.

2. The biodegradable non-woven fabric according to claim 1,
wherein the biodegradable binder is enriched in the entangling points of the non-woven fabric whereas the amount of the binder is lower in areas between the entangling points;
and/or
wherein covalent bonding points are created between the biodegradable fibers, in particular within entangled areas;
and/or
wherein the binder at least partially coats a surface of the biodegradable fibers especially in the entangled areas;
and/or
wherein the biodegradable binder further comprises glycerol or similar additives reducing the brittleness of the non-woven fabric.

3. The biodegradable non-woven fabric according to claim 1 or 2, wherein substantially all fibers comprised in the biodegradable non-woven fabric are biodegradable fibers.

4. The biodegradable non-woven fabric according to any one of the preceding claims, wherein the biodegradable fibers comprise cellulosic fibers, in particular regenerated cellulose fibers.

5. The biodegradable non-woven fabric according to claim 4, wherein the regenerated cellulose fibers are selected from the group consisting of viscose or lyocell.

6. The biodegradable non-woven fabric according to any one of the preceding claims, wherein the biodegradable fibers have an average fiber length of from 15 to 60 mm.

7. The biodegradable non-woven fabric according to any one of the preceding claims, wherein the biodegradable binder is a hydrophilic binder, in particular wherein the biodegradable binder is selected from the group consisting of chitosan, modified starch, cellulose derivatives, in particular blends of carboxymethylcellulose and citric acid, and protein based binders, such as casein.

8. The biodegradable non-woven fabric according to any one of the preceding claims, wherein the non-woven fabric has a basis weight of from 20 to 300 g/cm³.

9. The biodegradable non-woven fabric according to any one of the preceding claims, wherein the non-woven fabric does substantially not comprise thermoplastic non-biodegradable fibers.

10. The biodegradable non-woven fabric according to any one of the preceding claims, wherein the non-woven fabric is treated with a liquid or a lotion.

11. A method for producing a biodegradable non-woven fabric, comprising the steps of:
(a) forming a fibrous web comprising biodegradable fibers;
(b) entangling at least a part of the biodegradable fibers with each other by subjecting the fibrous web to a water-jet treatment or needle punching;
(c) applying a biodegradable binder to the entangled fibrous web; and
(d) drying the entangled fibrous web.

12. The method according to claim 11, wherein the drying comprises through-air drying.

13. A biodegradable non-woven fabric obtainable by a method according to claims 11 or 12.

14. A wipe comprising or consisting of the biodegradable non-woven fabric according to any one of claims 1 to 10 or 13.

15. The wipe according to claim 14,
wherein the wipe is a dry wipe or a wet wipe and/or
wherein the wipe is selected from the group consisting of facial wipes, cosmetic wipes, baby wipes, sanitary wipes, kitchen tissue, paper towel, handkerchiefs, cleaning tissue, cleansing tissue, floor mop and hard surface cleaning wipe.
